# EUROPEAN PATENT APPLICATION

(11) **EP 1 260 231 A1**
(43) Date of publication of application: **27.11.2002**
(21) Application number: 01908117.3
(22) Date of filing: 28.02.2001
(51) Int. Cl.: A61K 38/57, A61K 38/36, A61K 38/17, A61P 9/10, A61P 27/02, A61P 27/06

(54) **DRUGS FOR AMELIORATING RETINAL FUNCTION**

(30) Priority: 29.02.2000 JP 2000054322
(71) Applicant: TORAY INDUSTRIES, INC., Tokyo 103-8666 (JP)
(72) Inventor: MUTOH, Masato, Yokohama-shi, Kanagawa 240-0016 (JP); HOZUMI, Kaori, Ichikawa-shi, Chiba 272-0023 (JP); SUDO, Tetsuo, Fujisawa-shi, Kanagawa 251-0027 (JP); UTSUMI, Jun, Tokyo 131-0033 (JP)
(74) Representative: Kador & Partner
(86) International application number: JP0101476
(87) International publication number: WO01064242

(57) **Abstract**

The invention is a medicine for improving retinal functions comprising a protein that improves ERG (electroretinogram) recordings. Particularly, a medicine containing tissue-factor-pathway inhibitor-2 or placental protein 5 as the active ingredient can be used for ophthalmic diseases such as glaucoma, occlusion disease of the retinal and choroidal vessels and retinal dysfunctions, as a medicine for improving retinal functions.

## Description

### Technical Field

The present invention relates to a medicine for improving retinal functions, containing a protein that improves electroretinogram (ERG) recordings and which is useful clinically or experimentally as an active ingredient in medical drugs or reagents.

### Background Art

Vision is one of the most important senses and 80% of information in the external world is obtained through the visual system. Therefore, impairments of visual system such as reduced vision and loss of visual function are considered to be serious physical impairments. Particularly, considering the progressive aging society and today's information-centered society, it can be said that prevention of visual dysfunction is one of the important matters in the present medicine. Actually the importance of improvements in patients QOL for treatments of disorders that cause trouble in everyday life has been proposed recently. To establish the treatment methods for eye diseases and maintenance methods of visual system is particularly indispensable to the improvements in QOLV (quality of life and vision) and is an urgent necessity.

Serious reduced vision and loss of visual function may be due to various causes, and about 16,000 adults per year become blind in adulthood in Japan, the possible causes thereof including diabetic retinopathy, glaucoma, cataract, retinitis pigmentosa, and high myopia. As surgical treatments, photocoagulation by using lasers and vitreous surgery for diabetic retinopathy, trabeculectomy for glaucoma and insertion of intraocular lens for cataract are practiced. As medications, although various kinds of ophthalmic drugs to lower the intraocular pressure have also been developed for glaucoma, the results are not fully satisfactory still now. Pharmacotherapy has the advantage of being less invasive and simpler than invasive surgery, and is highly anticipated in the treatment of the increasing number of ophthalmic diseases, so developments in pharmacotherapy using suitable medicines are eagerly desired.

On the other hand, elucidation of pathology in ophthalmic diseases is also progressing because of developments in basic and clinical researches in recent years. That is, it has become clear that visual impairments result not only from lesions of visual cells of the retina, which is a sensorium in a narrow sense, but also from lesions of retinal pigment epithelium, which is greatly involved in the metabolism of the visual cells, impairments of nerve fibers, impairments of the retinal circulatory system, and furthermore, impairments of the choroidal system.

Among them, it is emerging that retinal pigment epithelial (RPE) cells play particularly important roles in maintenance of the visual cells. That is, the RPE cells play important functions physically and physiologically such as maintenance and regeneration of the visual cells: for example, they are arranged on Bruch's membrane, which is a lower layer in the retina, and absorb light which reaches to the retina to prevent reflection, and furthermore, they form a blood-retinal barrier which separates the visual cells and the choriocapillaris with the Bruch's membrane, and are also contribute to the production of various kinds of cytokines. The visual cells are protected by these RPE cells and Muller cells.

Moreover, important nerve modulating factors in the eye tissues include glutamic acid, acetylcholine, and dopamine, and on the other hand, interleukins, the TGFβ (transforming growth factor-β) superfamily, bFGF (basic fibroblast growth factor), VEGF (vascular endothelial cell growth factor), etc. are reported as the cytokines related to the RPE cells and are concerned with homeostasis in the eye (Makoto Tamai, Journal of Japanese Ophthalmological Society, 101, 889-890, 1997). However, it is still unknown whether these factors can be applied to useful treatments or not.

The present inventors have already confirmed that tissue-factor-pathway inhibitor-2 (TFPI-2) or placental protein 5(PP-5) as an active ingredient is effective in the ophthalmological treatments for retinal pigment degeneration, retinopathy, macular disorder, and retinal detachment (Japanese Patent Application No. 7-203304 and WO 97-05893).

It is known that TFPI-2 (Sprecher et al., Proc. Natl. Acad. USA, 91, 3353-3357, 1994) has inhibition activity for blood coagulation factor VIIa. Moreover, this was found to be a serine protease inhibitor produced by T98G glioma cells, and furthermore, it has been reported that the amino acid sequence thereof matches PP5 (Miyake et al., J. Biochem., 116, 939-942, 1994), which is concerned with blood coagulation. However, there are no reports until now whether these proteins have any activity to improve retinal function.

As described above, it is predicted in the future that ophthalmic diseases that cause serious reduction and loss of visual functions will increase. Suitable pharmacotherapy has still not been established, and most currently the research of cellular biology of the eye tissues has just started at last. There is no effective pharmacotherapy for the ophthalmic diseases mentioned above, and one of assignments of the treatments is TFPI-2 or PP-5 for improving the retinal degeneration.

Treatments for serious ophthalmic diseases, for example, surgery for lowering the intraocular pressure or administration of medicines is performed first for glaucoma, however, has been achieved definite success. It is emerging that there are limitations on recovering impaired vision and it is not possible to treat disease like normal tension glaucoma merely by the treatment for lowering the intraocular pressure. Moreover, there is also a problem in that occlusions of the central retinal artery and vein prevent visual acuity from fully recovering after restoring the circulatory impairments, and that secondary glaucoma develops.

With any of the above ophthalmic diseases, the retinal functions are severely impaired, which can cause serious visual dysfunctions such as reduced vision, visual field defects, and loss of visual functions. If there is an effective medicine which can be applied to these, it will clearly be a great contribution to practical medicine. The present invention relates to a medicine for improving retinal functions, containing a protein that improves electroretinogram (ERG) recordings and which is useful clinically or in research as an active ingredient in medical drugs or reagents.

### Disclosure of the Invention

The present invention relates to a medicine for improving retinal Functions, containing a protein, particularly, tissue-factor-inhibitor-2(TFPI-2) or placental protein 5(PP5). that improves ERG recordings and which is useful clinically or experimentally as an active ingredient in medical drugs or reagents.

### Brief Description of the Drawings

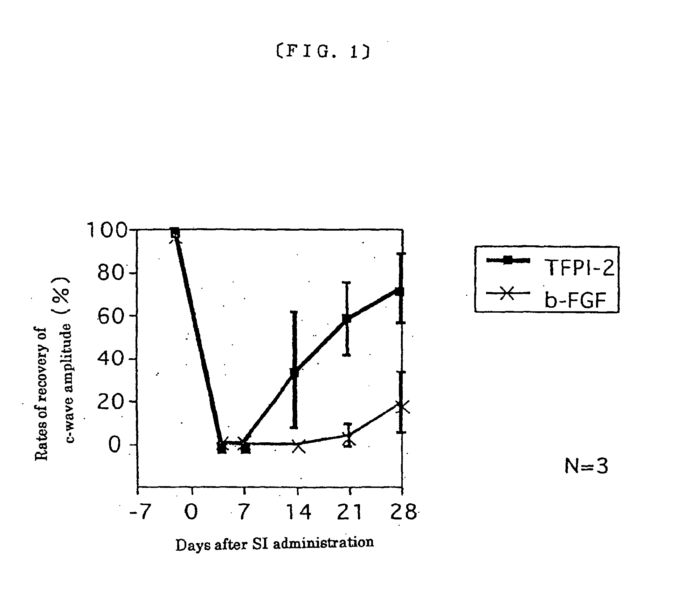

Fig. 1 shows improvement effects of ERG c-wave by TFPI-2 using SI-impaired rabbit models.

### Best Mode for Carrying Out the Invention

A factor is obtained by performing separation and purification from culture supernatants of human cultured cells, separation and purification of extracted solutions or cell culture supernatants of cells created by the so-called recombinant DNA technology using cDNA for the factor, or furthermore, separation and purification of body fluid components such as milk from so-called transgenic animals obtained from fetus embryos by injecting cDNA into a suitable vector system. Candidates for the human cultured cells are any of various kinds of normal tissue-derived cells or cell lines with the ability of producing the growth factor, and epithelial cells, stromal cells, and fibroblasts are preferred. Mammalian cells such as Chinese hamster ovary (CHO) cells and mouse C127 cells, insect cells such as silkworms and Mamestra brassicae, and microorganisms such as Escherichia coli, bacillus subtilis, and yeast can be used, when preparing the factor using gene manipulation technology. Furthermore, mice, rats, hamsters, rabbits, goats, sheep, pigs, cows, and the like can be used, when the hosts thereof are transgenic animals.

The factor prepared by the above methods may be isolated and purified from the cell culture supernatants, the insect extracts, the bacterial extracts, and the biogenic extracts used as a raw material, by various methods of chromatography. Any type of chromatography having affinity with the growth factor can be used: for example, columns using silicon dioxide (silica) or calcium phosphate as an adsorption material, columns with ligands of heparin, dye, or hydrophobic groups, metal chelate columns, ion exchange columns, gel filtration columns, and so on.

It is known that ERG used herein reflects the functionality of the RPE cells, which are indispensable to the survival of the visual cells existing in the retina, and it is known that the amplitude of ERG will be down or disappear when the functions of the RPE cells decrease, i.e., the retinal functions decrease with the ophthalmic disease accompanying the retinal degeneration or visual dysfunctions, specifically, the disorders such as glaucoma (primary glaucoma and secondary glaucoma), and occlusion disease of the retinal vessels (arteriolosclerosis, central retinal artery occlusion, central retinal vein occlusion, branch retinal vein occlusion) (Brown, K.T. et al. J. Physiol. 158, 257-280, 1961). No medicine showing an improvement in ERG recordings, i.e., an improvement of the retinal functions, has been found for the above described disorders accompanying retinal degeneration.

The inventors have confirmed that TFPI-2 or PP5 can recover c-wave amplitudes of ERG, improve the functions of the RPE cells, and prevent the impairments of the visual cells in drug-induced retina-injured models as shown in Examples.

As mentioned above, the inventors have found that the factor, that is, TFPI-2 or PP5 are useful clinically or experimentally as a medicine for improving the retinal functions or as a reagent, and are widely applicable as therapeutic agent which improve the visual functions in ophthalmic diseases accompanying retinal degeneration and visual dysfunction.

The factor can be administered directly or as a medical composition mixed with well-known pharmaceutically acceptable carriers, excipients, or the like, and orally or parenterally.

Specifically, formulations for oral administration include tablets, pills, capsules, pellets, syrups, emulsions, suspensions, and the like. These formulations are manufactured by well-known procedures and contain carriers or excipients usually used in a drug product field. For example, the carriers and the excipients for tablets can include lactose, maltose, saccharose, starch, magnesium stearate, and the like.

Formulations for the parenteral administration include, for example, salves, creams, injections, poultices, liniments, suppositories, ophthalmic solutions, transnasal medicine, inhalation, transdermal medicines, and the like. Particularly, the injections (systemic application, intravitreal administration, subretinal administration, Tenon's capsule administration, subconjunctival administration, and the like), kerato-conjunctiva medicines, the ophthalmic solutions, opthalmic ointments, arid the like are preferred for use in local ophthalmological administration. The solution formulations can be prepared by well-known procedures, for example, in general, by dissolving the factor in a sterile aqueous solution, suspending in an extract, or emulsifying and embedding in liposomes to be used for the injection. Drug formulations can be prepared by mixing an isotonizing agent (sodium chloride, potassium chloride, boric acid, glycerol, and the like), a buffer (a boric acid buffer solution, a phosphoric acid buffer solution, an acetic acid buffer solution, and the like), a solubilizer (surfactants and cyclodextrins), a stabilizing agent (citric acid, ethylenediaminetetraacetic acid, sodium sulfite, sodium bisulfite, and the like), and a viscous agent (synthetic macromolecules, celluloses, and polyalcohols). Furthermore, it is also possible to prepare in dosage forms such as high molecular polymer agents, microsphere preparations, sols, gels, and ointments.

Solid formulations can be prepared by well-known procedures, for example, by adding an excipient such as mannitol, trehalose, sorbitol, lactose, and glucose to the factor to provide lyophilized materials. Furthermore, this can be reduced to fine particles for utilization. Gelled agents can be prepared by well-known procedures, for example, by dissolving the factor in a thickener or polysaccharide, such as polyethylene glycol, methylcellulose, carboxyl methylcellulose, hyaluronic acid, or chondroitin sulfate.

In any of the formulations, stabilizing agents such as human serum albumin, human immunoglobulin, α2 macroglobulin, amino acids, and the like can be added, and dispersing agents or absorption accelerators such as alcohols, sugar alcohols, ionic surfactants, nonionic surfactants, and the like can be added in the range in which the physiological activities of this factor are not impaired. Moreover, trace metals and salts of organic acids can also be added if needed.

Administration of the medicine for improving retinal functions of the present invention is performed by systemic administration or local administration, the effective dose and the number of times of the administration will also change with administration dosage forms, administration routes, patients' ages, weights, disorders for treatment, symptoms, or severity of stage, and preferably 0.001-100 mg, more preferably 0.01-10 mg can be administered per adult once or several times.

### [Examples]

Hereinafter, Examples are shown in order to describe the invention in detail.

### EXAMPLE 1

### Pharmacological tests using RCS rats:

The protective effects of TFPI-2 on the retinal impairments were examined using RCS (Royal College of Surgeons) rats, which are known as being an animal model having a genetically impaired retina and retinal tissues and which start degenerating at around 25 days old (Ella, G. et al. Nature 347: 83-86, 1990). TFPI-2 was produced by the procedure described in "Invention of therapeutic agent containing TFPI-2 or PP-5 as active ingredient for retinal pigment degeneration and retinal a disorders" (Japanese Patent Application No. 7-203304 and WO 97-05893). It was administered into the vitreous body of male RCS rats anesthetized with ketamine/xylazine (ketamine:xylazine:physiological saline = 10:1:1) using a 30 gauge needle. TFPI-2 administration was performed twice: at three weeks old and at four weeks old. The administration volume per dose was 10 µl, and the total amount of proteins administered was 7 µg. PBS (phosphate buffer saline) was administered as a control. The eyeball was removed at five weeks old, and pathologic sections were prepared after fixing in glutaraldehyde/formalin by conventional methods. Staining was performed by the hematoxylin-eosin method. The results are shown in Table 1.

**Table 1**

| Retinal degeneration depression effects of TFPI-2 in RCS rats | | |
|---|---|---|
| | Pathological finding | |
| | PBS-treated group | TFPI-2-treated group |
| Outer nuclear layer | The number of cells constituting the layer decreased remarkably and a one-layer structure was formed. | A three-layer structure was maintained. |
| Rod-cone cell layer | Atrophy of cells constituting the layer and moderate vacuolation were observed. | Little atrophy of the cells was observed. Slight vacuolation was observed. |
| RPE cell | Little proliferation was observed. A small amount of degeneration was observed. | Little proliferation was observed. A small amount of degeneration was observed. |

It was observed in the PBS-treated group that the outer nuclear layer composed of visual cells decreased and the rod-cone cells atrophied with vacuole formation. In the TFPI-2-treated group, the reduction of the outer nuclear layer was suppressed and atrophy and vacuolation of the rod-cone cells were also suppressed. On the other hand, no particular proliferation was observed in the RPE cells compared with the PBS-treated group. The results show that the TFPI-2 protected the visual cells without stimulating the proliferation of the RPE cells, and thus the expected effects of the functions of the RPE cells being improved by the TFPI-2 administration were observed.

### EXAMPLE 2

### Pharmacological tests using sodium iodate (which is named SI hereinafter)-injured rabbit models:

The effects of improving the functions of the RPE cells by TFPI-2 were verified using the SI-injured rabbit model (Heike, M. et al. Docummenta Ophthalmologica 75: 15-22, 1990), which is a typical chemical-induced retina-injured model. SI (15 mg/kg) was administered intravenously to colored rabbits (Dutch rabbits), and TFPI-2 was administered into the vitreous body the next day. The administration volume per dose was 100 µl, and the total amount of proteins administered was 79 µg. The same protein amount of bFGF was used as a control. Then, the ERG c-wave was measured with time. The rabbits were left in a dark room for 1.5 hours or more to adapt to dark, and then anesthetized with ketamine/xylazine (5:3). The rabbits were fixed in the prone position, and treated with a mydriatic agent to produce mydriasis, and then, contact lens type electrodes were applied to them. Light stimulus was performed at 1000 lux for 5.5 seconds using a white LED electrode light-emitting device connected to an electrode. The ERG c-wave generated by the stimulus was amplified by a direct current amplifier, and was recorded on a memory oscilloscope. The measurements were performed several times every 5 minutes, and the wave forms were added and averaged to obtain the amplitudes. The amplitude at each point was compared with the normal amplitude before the SI administration, and the rates of recovery of c-wave amplitude were obtained. It has been reported that the ERG c-wave reflects the functions of the RPE cells (Steinberg, R. H. et al. Nature 227, 728-730 1970). The results of the Example are shown in Fig. 1. The TFPI-2-treated group showed clear effects of recovering the c-wave compared with the bFGF-treated group. After 28 days from the SI administration, compared with the normal amplitude, eight out of ten recovered in the TFPI-2-treated group, but only two out of ten recovered in the bFGF-treated group.

Examples 1 and 2 described above revealed that TFPI-2 clearly improves the functions of the RPE cells, and prevents impairments of the visual cells.

### Industrial Applicability

According to the present invention, a protein having the ability to improve ERG is effective as a treatment agent for retinal dysfunctions, specifically, ophthalmic diseases such as glaucoma and occlusion disease of the retinal and choroidal vessels.

## Claims

1. A medicine for improving retinal functions, comprising a protein that improves electroretinogram recordings.

2. A medicine for improving retinal functions according to Claim 1, wherein the protein that improves electroretinogram recordings is tissue-factor-pathway inhibitor-2.

3. A medicine for improving retinal functions according to Claim 1 or 2, wherein the medicine is for glaucoma or occlusion disease of the retinal and choroidal vessels.
